Europäisches Patentamt

European Patent Office

Office européen des brevets

(19)

(11) Publication number: **0 061 540**
**B1**

# EUROPEAN PATENT SPECIFICATION

(12)

(45) Date of publication of patent specification: **30.07.86**

(51) Int. Cl.⁴: **A 61 F 2/02, A 61 B 19/00**

(21) Application number: **81301337.2**

(22) Date of filing: **27.03.81**

(54) **Prosthesis for prevention of gastro esophageal reflux.**

(43) Date of publication of application:
**06.10.82 Bulletin 82/40**

(45) Publication of the grant of the patent:
**30.07.86 Bulletin 86/31**

(84) Designated Contracting States:
**AT BE CH DE FR GB IT LI LU NL SE**

(56) References cited:
**US-A-3 863 622**
**US-A-3 875 928**
**US-A-4 178 915**

(73) Proprietor: **Angelchik, Jean Pierre**
**1728 W. Glendale Avenue, Suite 401**
**Phoenix Arizona 85021 (US)**

(72) Inventor: **Angelchik, Jean Pierre**
**1728 W. Glendale Avenue, Suite 401**
**Phoenix Arizona 85021 (US)**

(74) Representative: **Newens, Leonard Eric et al**
**F.J. CLEVELAND & CO. 40/43 Chancery Lane**
**London WC2A 1JQ (GB)**

EP 0 061 540 B1

## Description

This invention relates to a surgical prosthesis.

More particularly, the invention concerns an improved prosthesis for use in preventing gastro esophageal reflux.

When the esophageal hiatus of the diaphragm muscle becomes enlarged, a portion of the stomach immediately below the gastro esophageal junction (the gastric fundus) may actually slide upwardly through the esophageal hiatus into the chest or thoracic cavity. This anatomic condition, known as a "sliding esophageal hernia" frequently causes gastro esophageal reflux in which stomach acids and foods are regurgitated into the esophagus. The characteristic symptoms of gastro esophageal reflux consist of substernal burning, regurgitation and frequent eructations. These symptoms are accentuated by recumbency, tight garments and physical activity, particularly bending at the waist. Long-term gastro esophageal reflux leads to complications, namely dysphagia from an esophageal stricture.

Various procedures have been devised for the repair of sliding esophageal hernias and for the prevention of gastro regurgitation and eructations. Crural repair almost invariably fails since it is almost impossible to effectively suture the constantly moving diaphragm. Therefore, other procedures to prevent the stomach from sliding through the enlarged esophageal hiatus were devised. According to the "posterior gastropexy" procedure of Hill, the crura is closed behind the esophagus and the stomach is sutured to the arcuate ligament over the aorta to hold the stomach within the abdominal cavity. According to the "Niessen II" procedure, the gastric fundus is formed into a ring around the distal esophagus. The added bulk of this ring around the esophagus forms a valve preventing regurgitation and, at the same time, preventing the stomach from sliding through the enlarged esophageal hiatus.

The Hill procedure described above is very difficult to perform on obese patients and it appears that the sutures from the lesser curvature of the stomach to the arcuate ligament are of a transient nature. According to the Niessen II procedure, it is necessary to suture the stomach to itself and to the esophagus.

My earlier issued U.S. Patent, No. 3,875,928, discloses a further method for maintaining the reduction of a sliding esophageal hiatal hernia. The method comprises emplacing a C-shaped prosthesis about the distal esophagus between the gastric fundus and the diaphragm. The prosthesis has a tape secured about the periphery thereof. The free ends of the tape, which extend well beyond the tapered ends of the C-shaped cushion, are tied together then sutured to the stomach to maintain the prosthesis in proper operative position.

Both the Hill and Niessen II procedures described above and the method described in my earlier issued U.S. Patent No. 3,875,928, attempt to maintain the stomach in its natural position below the diaphragm in order to prevent gastro esophageal reflux.

As illustrated in Figure 6 of my earlier issued patent, when the prosthesis is in the proper operative position, the larger diameter central portion of the C-shaped cushion bears against the hind side of the esophagus 17 and accentuates the curvature which exists in the portion of the esophagus between the stomach 43 and diaphragm 13. This increased curvature in the esophagus 17 apparently minimizes the probability of recurrence of gastro esophageal reflux and also makes upward migration of the prosthesis and upper portion of the stomach 45 through the hiatal hernia more difficult.

Observation has confirmed that the repetitive contracting movements of the stomach 43 cause the sutures attaching free ends 27 to the stomach 43 to be of transient nature. When the sutures free themselves from the wall of the stomach 43, the prosthesis 20 tends to rotate about the esophagus 17 and the tapered ends 23 move behind the distal esophagus 17 and above the upper part of the stomach 45. When the tapered ends 23 have rotated to a position behind the esophagus 17, outward pressure on the esophagus 17 is largely diminished. As a result, the additional curvature originally imparted to the esophagus by the greater sized center portion of the prosthesis is lost, and the recurrence of gastro esophageal reflux is more probable.

In addition, after rotating to a position above the upper part of the stomach 45, the tapered ends 23 typically move upwardly into and through the enlarged esophageal hiatus 12 such that the prosthesis 20 migrates from the desired operative position to a position above the diaphragm 13.

Alternatively, when the sutures disengage from the stomach 43, the prosthesis 20 may, without rotating, simply travel essentially vertically up the esophagus 17 allowing the tapered ends 23 to move into the enlarged esophageal hiatus 12 and promote migration of the prosthesis from the proper operative position to a position above the diaphragm 13.

Accordingly, it is a principle object of the present invention to provide an improved prosthesis for preventing gastro esophageal reflux.

Another object of the invention is to provide an improved prosthesis for maintaining the intra-abdominal reduction of a sliding esophageal distal hernia.

According to the present invention there is provided a prosthesis (20) for use in maintaining the intro-abdominal reduction of a sliding oesophageal hiatal hernia by preventing extension of the gastric fundus into the thoracic cavity through an enlarged oesophageal hiatus, said prosthesis comprising:—

(a) a generally C-shaped cushion member (21), the inside diameter of which generally corresponds to the normal outside diameter of the distal oesophagus (17) and the outside diameter thereof being larger than said enlarged oesophageal hiatus, said C-shaped member (21) being deformable to permit adjustment of the spacing of the free ends (23) thereof and having a generally constant cross-sectional area along the length thereof; and

(b) tie means (26, 27) for securing said cushion member (21) in its operative configuration around the distal oesophagus (17) between the gastric fundus (45) and the diaphragm (13) so that said ends are in closely spaced relationship and without the need for suturing.

The invention will now be described by way of example only with particular reference to the accompanying drawings wherein:

*Figure 1* is a perspective view of the preferred embodiment of the prosthesis which I use in accordance with my improved procedure for repairing a sliding esophageal distal hernia;

*Figure 2* is a sectional side view of the prosthesis of Figure 1 taken along section line 2—2 thereof;

*Figure 3* is a top view of the prosthesis of Figure 1;

*Figure 4* is a perspective anatomical drawing illustrating the initial steps according to my procedure of reduction of the hiatal hernia;

*Figure 5* is a perspective anatomical drawing illustrating the prosthesis of Figures 1—3 located in a proper operative position to prevent recurrence of the hiatal hernia; and

*Figure 6* is a cross sectional view of the stomach, diaphragm and esophagus illustrating a typical sliding esophageal hernia.

Briefly, I provide an improved prosthesis for use in maintaining the intra-abdominal reduction of a sliding esophageal hiatal hernia by preventing extension of the gastric fundus into the thoracic cavity through an enlarged esophageal hiatus. The prosthesis comprises a generally C-shaped cushion member, and tie means for securing said cushion member in its operative configuration around the distal oesophagus between the gastric fundus and the diaphragm so that said ends are in closely spaced relationship and without the need for suturing.

The C-shaped cushion is deformable to permit adjustment of the spacing of the free ends thereof at a selected distance and has a generally constant cross-sectional area along the length thereof. The inside diameter of the prosthesis generally corresponds to the normal outside diameter of the distal esophagus and the outside diameter thereof is larger than the enlarged esophageal hiatus.

The prosthesis can be used to prevent gastro esophageal reflux when the gastro esophageal junction is positioned above the diaphragm.

The generally C-shaped cushion prosthesis is positioned around the distal esophagus, the free ends of the prosthesis are adjusted to a spacing to permit normal expansion of the esophagus during swallowing, and the free ends of the prosthesis are secured in the adjusted position to maintain the C-shaped cushion prothesis in operative configuration around the distal esophagus generally adjacent to the gastric fundus when the gastro esophageal junction is positioned above the diaphragm and to maintain the adjusted spacing of free ends. The C-shaped cushion has an inside diameter generally corresponding to the normal outside diameter of the distal esophagus, and is deformable to permit adjustment of the spacing of the free ends thereof at a selected distance.

The prosthesis can bear radiopaque indicia which facilitate radiographic determination of the position of the prosthesis after emplacement thereof around the distal esophagus. The means for maintaining the cushion member in its operative position preferably comprises an elongate tape member secured around the periphery of the cushion member. The free ends of the tape extend substantially beyond the free ends of the C-shaped cushion member, i.e., a distance sufficient to allow the ends of the tape member to be tied together.

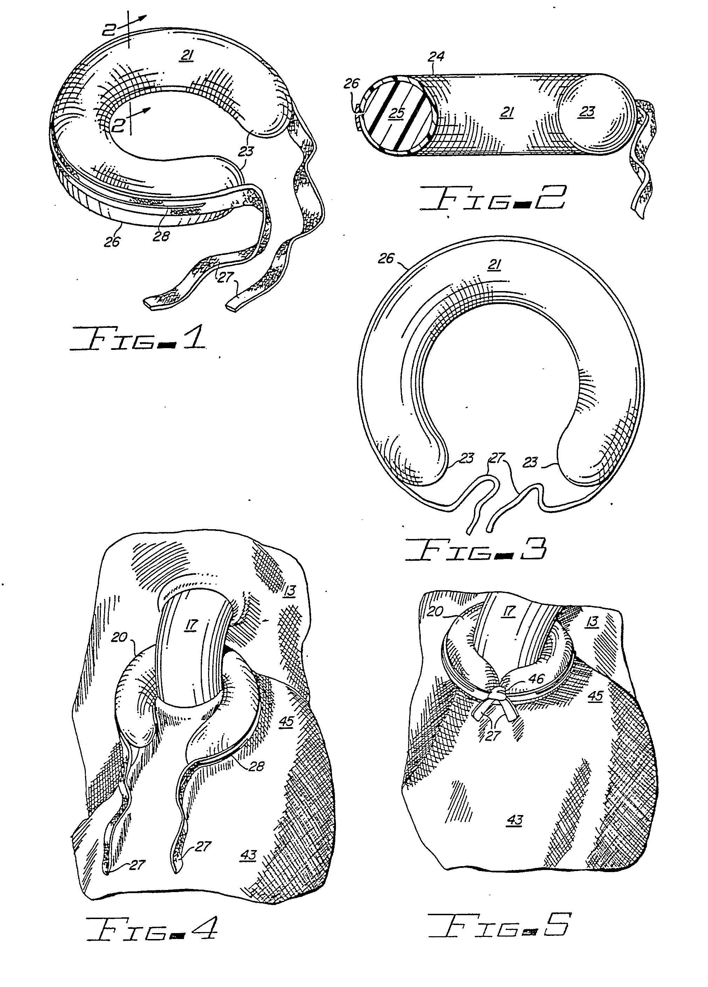

Turning now to the drawings, Fig. 1 depicts the preferred embodiment of the surgical prosthesis which I utilize to maintain the reduction of an esophageal hiatal hernia. The prosthesis consists of a generally C-shaped cushion member 21, the inside dimensions of which generally correspond to the normal outside dimensions of the distal esophagus (reference character 17, Fig. 5). In a typical prosthesis, the inside dimensions will equal about 3.1 by 2.5 centimeters, although prosthetic devices having somewhat larger and somewhat smaller inside dimensions should be provided to the surgeon for use where the patient may have an esophagus somewhat larger or somewhat smaller than normal.

The outside dimensions of the prosthesis are sized to be substantially larger than the enlarged esophageal hiatus (reference character 12, Fig. 5), and in a typical prosthesis, the outside dimensions will equal about 6.0 by 7.0 centimeters. Obviously, these outside dimensions are also variable in accordance with the size of the enlarged esophageal hiatus of a particular patient.

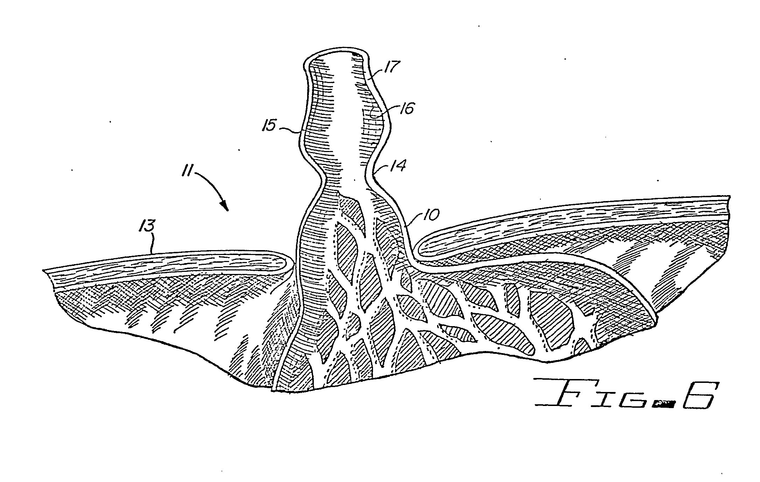

In accordance with the prosthesis as shown in Figs. 1—3, the cushion member has a generally circular cross-section and is of generally constant cross-sectional area along the length thereof. The prosthesis is preferably constructed by filling outer flexible integement 24 with a gel liquid 25 such that the entire cushion member 21 is deformable to permit adjustment of the spacing of the free ends 23 at a selected distance which will permit the normal expansion of the esophagus during swallowing. The precise materials of construction of the integement 24 and the filler 25 of the C-shaped cushion member 21 are not highly critical so long as they are compatible with body tissues, i.e., do not induce rejection or cause other body reaction. In the presently preferred embodiment of the invention, I employ a silicone elastomer shell filled with a highly cross linked silicone gel manufactured by Dow Chemical Company and sold under the trade name "Silastic". A tape 26, preferably silicone-coated Dacron, is secured to the C-shaped cushion member 21 around the outer periphery thereof and the free ends 27 of the tape extend substantially beyond the free ends 23 of the C-shaped cushion member 21 to a distance sufficient to allow the free ends 27 of the tape 26 to be tied together.

Preferably, the prosthesis is provided with a tantalum filled silicone strip 28 on tape 26 such that after implantation of the prosthesis, radiographic examination will reveal whether the prosthesis is in its proper operative position.

The method of use of the prosthesis of Figs. 1—3 is illustrated in Figs 4 and 5. My procedure consists of opening the abdominal cavity with an upper midline incision and exposing the area of the diaphragmatic hiatus by medially retracting the left lobe of the liver. The peritoneum and sac of the hiatal hernia 12 are then incised and the hernia 12 is reduced by retracting the stomach 43 intra-abdominally. As shown in Fig. 4, the prosthesis 20 of Figs. 1—3 is then placed around the distal esophagus 17 immediately above the gastric fundus 45. Fig. 5 illustrates the prosthesis 20 in position after the free ends 27 of the tape 26 are anteriorly tied at 46 to locate the free ends 23 of the prosthesis 20 at the proper spacing and the tape end remnants are cut leaving about an inch of length. Suturing of the free ends to the stomach or to other anatomical structures is not required to maintain the prosthesis 20 in its proper operative position around the distal esophagus 17 between the gastric fundus 45 and the diaphragm 13.

The prosthesis shown in my earlier issued U.S. Patent, No. 3,875,928, has tapered ends and a larger sized center portion. In the proper operational position, the center portion of the tapered prosthesis is situated behind the distal esophagus 17 to increase the curvature of the esophagus. Increased curvature of the esophagus apparently tends to reduce gastro esophageal reflux. When the sutures binding the free ends of the Dacron (Trade Mark) tape to the stomach free themselves the tapered ends of the C-shaped cushion prosthesis tend to rotate to a position behind the esophagus 17 and above the gastric fundus 45. Since the size of the tapered ends of the prosthesis is insufficient to accentuate the curvature of the esophagus, the lessening of gastro esophageal reflux achieved when the tapered prosthesis also initially placed in the proper operative position is lost. In addition, after rotating to a position above the gastric fundus, the tapered ends tend to enter the hiatal hernia 12 and promote upward migration of the prosthesis through the diaphragmatic hiatus 12.

In contrast to the tapered prosthesis described in my earlier issued patent, the prosthesis herein illustrated in Figures 1—3 is of generally constant cross-sectional area along its entire length, i.e., the ends of the prosthesis are not substantially tapered. Consequently, even if the prosthesis rotates about the distal esophagus, the accentuation of the curvature of the esophagus 17 is maintained. Further, in the great majority of hiatal hernias, the consistent size of the present prosthesis along the length thereof eliminates upward migration of the prosthesis into and through the diaphragmatic hiatus. The prosthesis generally maintains the proper operative position between diaphragm 13 and stomach 43.

In sum, the present prosthesis need not be sutured in position to effectively maintain the reduction of a sliding esophageal hiatal hernia.

Figure 6 depicts a typical sliding esophageal hiatal hernia and shows the gastric fundus 10 extending into the thoracic cavity 11 through the enlarged esophageal hiatus 12 of the diaphragm 13. In this position, the lesser sphincter 14 of the esophagus 15, being transposed into the chest from its normal position just below the esophageal hiatus 12 operates less effectively. This permits gastro esophageal reflux of stomach acids and foods which are not evacuated by esophageal peristalsis and which remain in the lower esophagus for prolonged periods causing irritation and damage to the lower esophagus mucosa 16.

In order to prevent gastro esophageal reflux, the method described in my earlier issued U.S. Patent No. 3,875,928, attempts to preclude upward migration of the stomach through the esophageal hiatus by suturing a prosthesis emplaced around the esophagus to the stomach. As described herein, the present prosthesis can be used, without suturing whatsoever and without reduction of the hiatal hernia, to prevent gastro esophageal reflux by placing it around the esophagus when the gastro esophageal junction is located above the diaphragmatic hiatus.

### Claim

A prosthesis (20) for use in maintaining the intra-abdominal reduction of a sliding oesophageal hiatal hernia by preventing extension of the gastric fundus into the thoracic cavity through an enlarged oesophageal hiatus, said prosthesis comprising:—

(a) a generally C-shaped cushion member (21), the inside diameter of which generally corresponds to the normal outside diameter of the distal oesophagus (17) and the outside diameter thereof being larger than said enlarged oesophageal hiatus, said C-shaped member (21) being deformable to permit adjustment of the spacing of the free ends (23) thereof and having a generally constant cross-sectional area along the length thereof; and

(b) tie means (26, 27) for securing said cushion member (21) in its operative configuration around the distal oesophagus (17) between the gastric fundus (45) and the diaphragm (13) so that said ends are in closely spaced relationship and without the need for suturing.

### Revendication

Prothèse (20) à utiliser pour maintenir la réduction intra-abdominale d'une hernie hiatale oesophagienne glissante par prévention de l'extension du fond gastrique dans la cavité thoracique à travers un hiatus oesophagien élargi, ladite prothèse comprenant:

(a) un organe formant coussin (21) généralement en forme de C, dont le diamètre intérieur correspond généralement au diamètre extérieur normal de l'oesophage distal (17) et dont le diamètre extérieur est plus grand que ledit hiatus oesophagien élargi, ledit organe (21) en forme de C étant déformable pour permettre l'ajustement de l'espace entre ses extrémités libres (23) et ayant une aire en coupe transversale généralement constante sur sa longueur; et

(b) un moyen d'attache (26, 27) pour fixer ledit organe formant coussin (21) à sa configuration active autour de l'oesophage distal (17) entre le fond gastrique (45) et le diaphragme (13) de façon que lesdites extrémités soient en relation très peu espacée et sans nécessiter de suture.

**Patentanspruch**

Prothese (20) zur Verwendung bei der Aufrechterhaltung der intra-abdominalen Reduktion einer Gleithernie durch den Hiatus oesophageus durch Verhinderung der Ausdehnung des Magengrundes in die Brusthöhle durch einen vergrößerten Hiatus oesophageus hindurch, gekennzeichnet durch:

a) ein im wesentlichen C-förmiges Kissenelement (21), dessen Innendurchmesser im wesentlichen dem normalen Außendurchmesser der distalen Speiseröhre (17) entspricht und dessen Außendurchmesser größer als der vergrößerte Hiatus oesophageus ist, wobei das C-förmige Element (21) deformierbar ist, um die Einstellung des Abstandes zwischen seinen freien Enden (23) zu ermöglichen, und eine im wesentlichen konstante Querschnittsfläche über seine Länge hin aufweist; und

b) Bandmittel (26, 27) zur Sicherung des Kissenelementes (21) in seiner Einsatzkonfiguration um die distale Speiseröhre (17) zwischen dem Magengrund (45) und dem Zwerchfell derart, daß die freien Enden eng beieinander liegen, ohne vernäht werden zu müssen.

0 061 540

FIG-1

FIG-2

FIG-3

FIG-4

FIG-5

1

Fig. 6